# EUROPEAN PATENT APPLICATION

(11) **EP 4 023 060 A1**
(43) Date of publication of application: **06.07.2022**
(21) Application number: 20217492.6
(22) Date of filing: 29.12.2020
(51) Int. Cl.: A01K 67/033

(54) **SYSTEMS FOR REARING INSECTS AND METHOD FOR OPERATING AN INSECT REARING SYSTEM**

(71) Applicant: Bühler AG, 9240 Uzwil (CH)
(72) Inventor: JANSEN, Maurits Petrus Maria, 4854 CA Bavel (NL); GILLIS, Jacobus Henricus Antonius Maria, 4835 BG Breda (NL); HARMS, Stijn, 5212 AM Hertogenbosch (NL); DE GELDER, Vincent, 4207 VC Gorinchem (NL)
(74) Representative: Vossius & Partner Patentanwälte Rechtsanwälte mbB

(57) **Abstract**

The invention provides a system for rearing insects, comprising at least one room (1) for storing a plurality of crates for storing insects to be reared, a dosing station (5) for filling insects and/or feed to the crates, an automated central harvesting unit (3) including a harvesting station (7) for unloading the insects from the crates for subsequent processing of the insects and/or a plurality of further processing stations, a crate collection system (2) for transferring the crates between the at least one room and the central harvesting unit comprising a plurality of crate collection devices (21) for collecting crates in or from the at least one room, and a connection (4) for connecting the at least one room and the central harvesting unit. The connection may be any means for transferring the crates between the at least one room and the central harvesting unit, including a path for allowing an automated guided vehicle to move from the at least one room to the central harvesting unit, a conveyer line connecting the at least one room to the central harvesting unit, a robot arm etc. The crates are used in a continuous loop in the system. The invention further provides a system for rearing insects, comprising at least one room, a compartment (11) or compartments of a room and/or a lane (12) for storing a plurality of crates for storing the insects to be reared, at least one swap area, leaving space for crates to be temporarily stored, and a collection system (2) for transporting the crates between rooms, compartments, and/or lanes and the swap area or vice versa. Moreover, the invention provides a method for operating an insect rearing system, in particular the systems according to the invention. That is, the system may comprise at least one room for storing a plurality of crates for rearing the insects, a dosing station for filling insects and/or feed to the crates, a central crate harvesting unit including a harvesting station for unloading the insects from the crates for subsequent processing of the insects and/or comprising a plurality of processing stations, and a plurality of crate collection devices for collecting crates in or from the at least one room. The crates may be transferred between the at least one room and the central harvesting unit via a connection.

## Description

The present disclosure relates to a system and method for rearing insects. In particular, the present disclosure relates to a system and method for rearing insects utilising a centralised harvesting unit for providing a continuous loop production cycle.

Production facilities for rearing insects usually require a large amount of manual labour or have various sub-stations which are interconnected by a complex logistics system. Depending on the type of insect, certain parameters like feedstock and climate have to be monitored and the facility has to be cleaned periodically. Current systems use multiple disconnected rooms and/or compartments with different climates for different age larvae. The larvae are grown in crates. The crates are removed regularly from the room for example in order to perform an operation like feeding or harvesting the insects or to clean the rooms. After performing such an operation, the crates return to a room or compartment. In current systems the logistics between the rearing or breed rooms and a processing section is largely performed manually. This may lead to downtimes of the rearing facility and thus to reduced output.

Rearing insects needs to be done in a controlled manner so that the outflow of product is consistent to reduce the load on equipment and logistic systems further down the line. The system needs to be able to run continually and not stop for cleaning. The system should also protect against excessive cross contamination between the insects. The present invention has been made in view of the above problems.

The invention provides a way of connecting at least one rearing room together with a central harvesting unit and/or swap area comprising at least two crate collection devices. This allows a constant flow of product from the at least one rearing room to a central harvesting area while allowing all areas of the rearing room to be cleanable without stopping the production. The room may be subdivided in separate compartments. The compartmentalisation protects the different zones of the room from cross contamination. At most, a single room may be lost if a disease, invasive insect species or parasitic outbreak occurs. Failures in certain equipment in the rearing system such as dosing stations will have reduced downtime as all crates can be redirected to any dosing station due to the interconnectivity.

In particular, the present disclosure relates to a system for rearing insects, comprising at least one room for storing a plurality of crates for storing insects to be reared, a dosing station for filling insects and/or feed to the crates, an automated central harvesting unit including a harvesting station for unloading the insects from the crates for subsequent processing of the insects and/or a plurality of further processing stations, and a crate collection system for transferring the crates between the at least one room and the central harvesting unit comprising a plurality of crate collection devices for at least collecting crates in or from the at least one room, and a connection for connecting the at least one room and the central harvesting unit. The connection may be any means for transferring the crates between the at least one room and the central harvesting unit, including a path for allowing an automated guided vehicle to move from the at least one room to the central harvesting unit, a conveyor line connecting the at least one room to the central harvesting unit, connection lines with automated crate handling devices like a shuttle, a shuttle-satellite, and/or a robotic arm etc. The crates are used in a continuous loop in the system.

The at least one room may be subdivided in separate compartments. The system may further comprise a climate system adapted to individually control the climate conditions for the at least one room and/or each compartment.

The central harvesting unit may further include, as a further processing station, a washing station for cleaning and, preferably, for drying empty crates and/or a weighing station for measuring the weight of a crate. The central harvesting unit is preferably followed by a separation process to separate insects from rearing residue.

The central harvesting unit preferably comprises a bypass for allowing an individual crate on the central harvesting unit to bypass one or several of the processing stations.

The crate collection devices of the collection system may directly or indirectly connect the at least one room with at least one of the processing stations. The connection between the at least one room and/or central harvesting unit and/or processing stations may be stationary or mobile as the crate collection devices may use automated guided vehicles for transporting individual crates or stacks of crates.

The transporting or the transferring of the crates by or along the connection is preferably automated.

"Stationary" means in this sense for example a fixed arranged pathway or arrangement of means along which the crates are transferred or transported, as for example rails or conveyers. Instead "mobile" means for example a flexible pathway on the ground.

The system may further comprise a controller for controlling rearing of insects in individual crates or sets of crates by automatically controlled transporting of the crates or sets of crates. The transport may include a transport between the at least one room and the central harvesting unit and the processing stations, the controller being configured such that the system provides for a regular output of reared insects.

The invention further provides a system for rearing insects, comprising at least one room, at least one compartment of a room and/or at least one lane for storing a plurality of crates for storing the insects to be reared, at least one swap area, leaving space for crates to be temporarily stored, and a collection system for transporting the crates between rooms, compartments, and/or lanes and the swap area or vice versa.

The swap area may be provided in at least one of the compartments and/or adjacent to the at least one room and/or within the reach (operating distance) of the crate collection system.

The swap area may have a size for storing crates of an entire lane, of a compartment and/or of a room.

Moreover, the invention provides a method for operating an insect rearing system, in particular the system according to the invention. That is, the system may comprise at least one room for storing a plurality of crates for rearing the insects, a dosing station for filling insects and/or feed to the crates, a central crate harvesting unit including a harvesting station for unloading the insects from the crates for subsequent processing of the insects and/or comprising a plurality of further processing stations, and a crate collection system for transferring the crates between the at least one room and the central harvesting unit comprising a plurality of crate collection devices for collecting crates in or from the at least one room. The crates may be transferred between the at least one room and the central harvesting unit via a connection.

For operating the system, a path of the crates through the rearing system is automatically individually controlled to
fill insects and/or feed to the crates,
store the crates in the at least one room, thereby rearing the insects, and
harvest the insects by unloading the insects and the feedstock residue from the crates for subsequent processing of the insects. The path of the plurality of crates through the system is controlled in such a way as to provide a regular output of reared insects.

The crates may be used inside the system in a continuous loop.

The method may further comprise a step of washing and drying the crates and/or weighing after harvesting the insects.

The crates may be automatically transported between the processing stations performing the respective method steps, between the at least one room and the central harvesting unit and/or between the plurality of crate collection devices by using a connection such as conveyor belts or automated guided vehicles.

The invention is further described in more detail with reference to the figures. Therein,
Fig. 1 shows a schematic overview according to an example of the present disclosure,
Figs. 2 to 5 show an overview of a system according to an example of the present disclosure,
Fig. 6 shows a connection according to an example of the present disclosure,
Fig. 7 shows an overview of the central harvesting unit according to an example of the present disclosure; and
Fig. 8 shows a swap area according to an example of the present disclosure.

Unless indicated otherwise, the same reference numerals denote the same or similar elements. The examples shown in the figures below are compatible with each other. The terms "central handling unit" and "central harvesting unit" refer to the same element and may be used interchangeably throughout the specification.

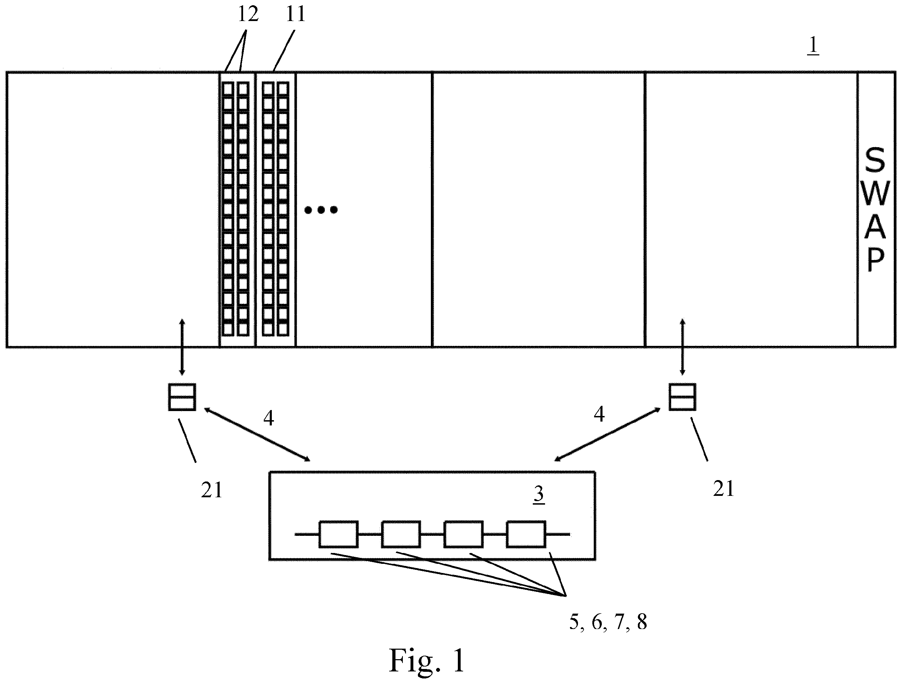

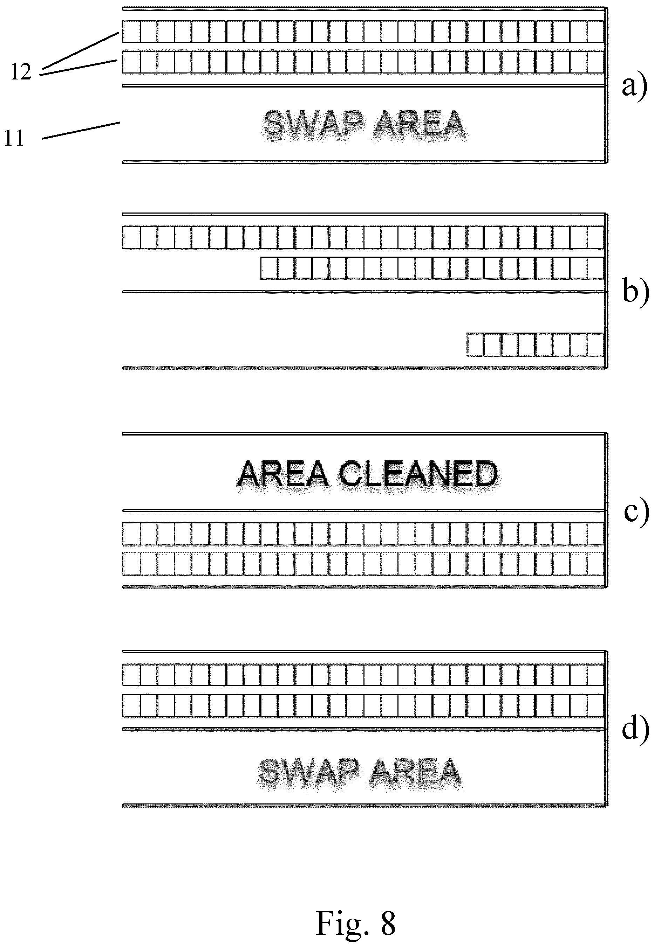

Fig. 1 provides a schematic overview of a basic configuration of a system according to the present disclosure. Therein, a room 1 having a plurality of compartments 11 each having a plurality of lanes 12 is shown. Also, a swap area is provided as indicated in the figure. The swap area will be described in detail below with reference to Fig. 8. A crate collection system comprising at least two collection system devices 21 or crate collection devices may be provided to move crates or stacks of crates between compartments 11, lanes 12, central harvesting or handling unit 3, connection station 4, processing stations 5, 6, 7, 8, to be described below and the swap area. All movement of crates may also be performed by automated guided vehicles. In other words, the collection system devices 21 may also form part of a central handling unit 3. There may also be a connection between the collection devices 21 and the central handling unit 3 or the processing stations. The elements present in the basic configuration of Fig. 1 will be described in more detail below.

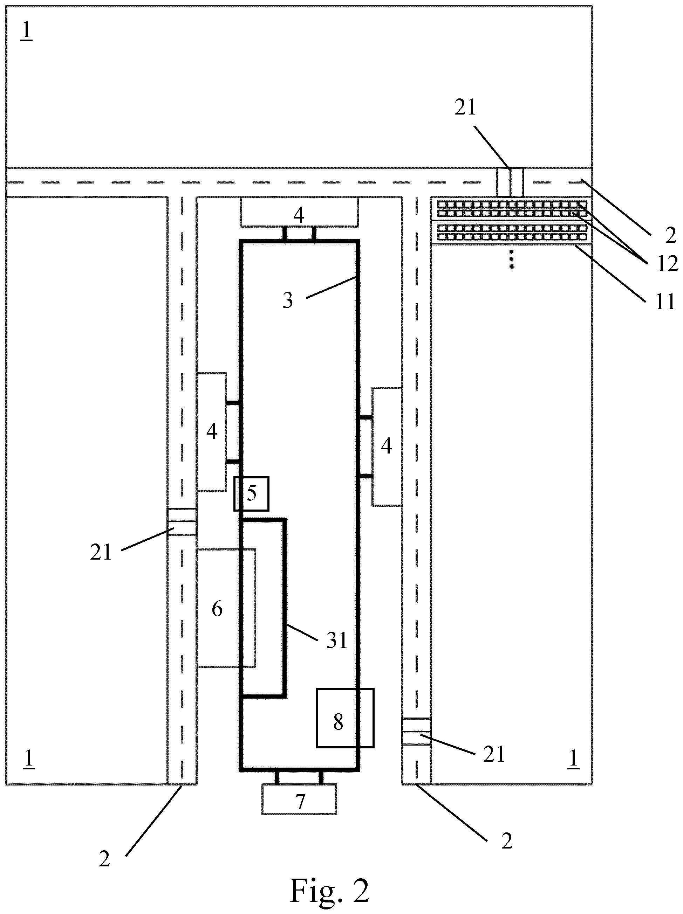

Fig. 2 shows an example of the present disclosure according to which the principles of the invention will be explained. It is, however, not to be understood as limiting. The system shown in Fig. 2 comprises three separate rooms 1 which may also be referred to as rearing rooms. The present disclosure is not limited to three rooms 1 but may also comprise one room 1 or a plurality of rooms 1. Each of the rooms 1 may be subdivided in compartments 11 separated by walls. Thereby, e.g. different climate conditions may be provided to each of the compartments or even to individual crate stacks or rows if desired. This is particularly useful if different kinds of insects and/or different development stages of insects are reared inside the facility. This also helps in providing a regular output of insects. Each compartment comprises at least one lane 12 of stacked crates for storing crates for rearing insects. Fig. 2 shows two lanes 12 per compartment 11, but one or a plurality of lanes 12 for storing crates for rearing insects may be provided.

Each room 1 is accessible via a collection system device 21 forming part of a collection system 2. The collection system 2 may also be understood as a logistics system or a logistics aisle. The device 21 may e.g. be an automated forklift or any other transport or conveying means for extracting one or a plurality of crates from the room 1 or compartments 11 or lanes 12. A single or plurality of collection system devices 21 may service one or a plurality of rooms 1. I.e., depending on the configuration, a single collection system device 21 may serve a plurality of rooms 1 or a plurality of collection system devices 21 may serve a single room 1. According to the invention as defined in claim 1, at least two collection devices may be provided.

From the crate collection devices 21 the crates are transferred via a connection station 4 to the central handling unit 3. Fig. 2 shows an individual connection station 4 for each room 1. However, the crate collection devices 21 may also do the connection to the central handling unit directly. Fig. 2 also shows a fixed connection for the transfer of the crates between the room 1 and the central harvesting unit 3 as the crates move along a fixedly installed means like rails or conveyors.

The system of Fig. 2 further comprises a central handling unit 3. The central handling unit 3 is configured to move crates inside the rearing facility between different processing stations 5, 6, 7, 8. The central handling unit 3 may transport single crates or stacks of crates. The central handling unit 3 is designed in such a way that it can service all processing stations 5, 6, 7, 8 either directly or through a connection station 4. The central handling unit 3 may consist of an automated conveyor system or logistics system which may be formed as a conveyor belt, automated guided vehicles (AGV) or any other means of transportation suitable for moving one or a plurality of crates. If automated guided vehicles are used, they can also directly access the rooms 1 without the collection system 2. In other words, the same automated guided vehicles can be used to collect the crates and then transport it from processing station to processing station.

The central handling unit 3 is also provided in such a way that each of the connection stations 4 can be bypassed. The movement direction of the crates is not fixed and may be changed at any time according to the rearing requirements. The central handling unit 3 is further connected to at least one filling station 5 or dosing station for filling feed and/or insect larvae into the crates. In addition or alternatively, feedstock may be provided to the crates inside the connection station 4. The system may further comprise a weighting station for determining the total weight of the crates and/or the weight of the added feedstock and/or insects. The weighting station may be provided separately or may be included in any one of the processing stations.

Depending on the insect type reared and its specific requirements, the crates are collected via the collection system 2 and transferred to the central handling unit 3 for re-feeding at the filling station 5 or directly at the connection station 4, or unloaded at the harvesting station 7. I.e., re-feeding may be performed through a crate collection device such as a collection system device 21, in a connection line between the room 1 and the processing stations, in the central handling unit 3 or at a separate re-feeding station.

In addition to the harvesting station 7, a separate rejection station 8 may be provided. There, crates which do not meet the required quality standards (i.e. weight) can be 'parked' to be inspected by an operator or automatically checked. Furthermore, contaminated crates may be emptied at the rejection station 8. In the central handling unit 3 a device may be foreseen to separately harvest crates not meeting the quality requirements.

A washing station 6, which can be bypassed via the bypass or re-batch line 31, is provided for cleaning used crates before (re-)filling. All processing stations 5, 6, 7, 8 can be bypassed through a bypass line 31 .

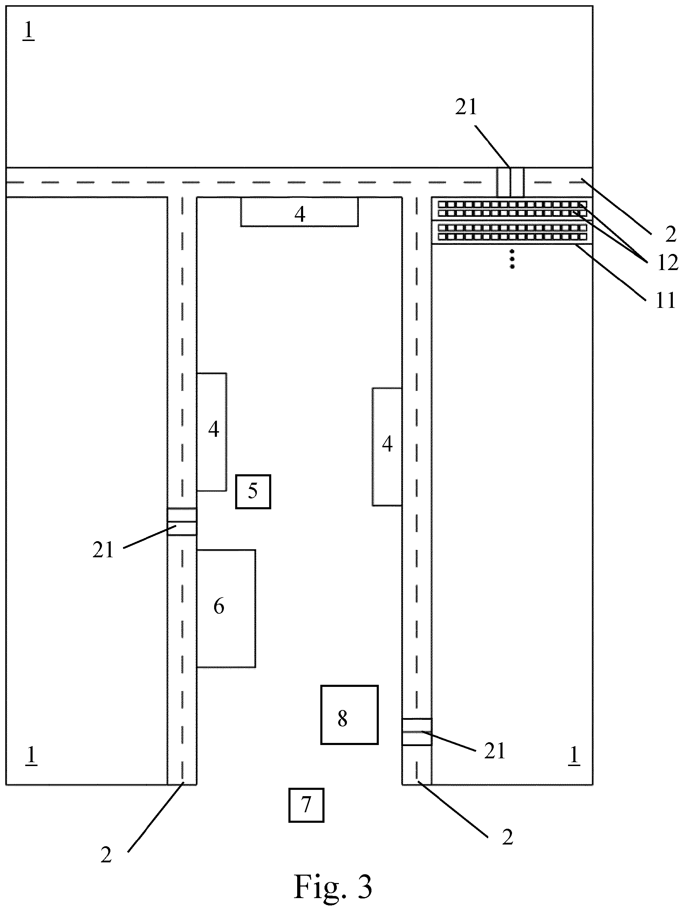

Fig. 3 substantially shows the same configuration as Fig. 2. A detailed description will thus be omitted. Fig. 3 renders evident that the central handling unit 3 does not need to move on fixed paths or in a fixed direction, particularly if automated guided vehicles are employed. These vehicles may, e.g. with the use of a collision control, move freely inside the facility and even connect room(s) 1, compartments 11 or lanes 12 with processing stations 5, 6, 7, 8 directly. They may also use predefined paths. Furthermore, the automated guided vehicles may be provided in addition to the collection system devices 21 or substitute the collection system devices 21 and perform all movement of the crates inside the facility.

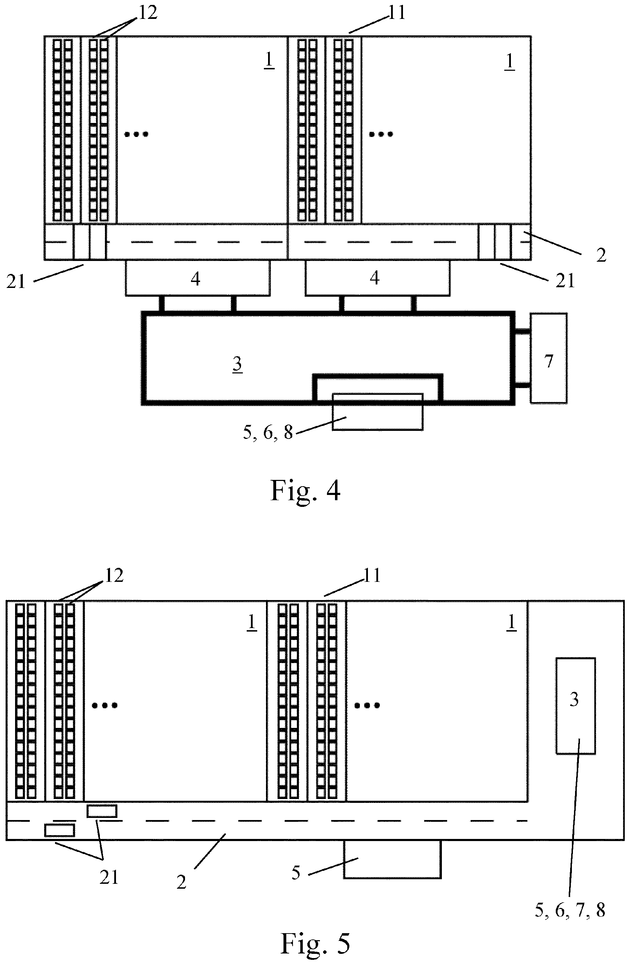

Fig. 4 shows a linear configuration of a system of the present disclosure, similar to one of the previous figures. That is, the rooms 1, in this case two rooms 1, are linearly arranged. The rooms 1 comprise compartments 11 and lanes 12 as described above. Furthermore, connection stations 4 and other processing stations 5, 6, 7, 8 may be provided as single or combined processing stations. The collection system devices 21 of the collection system 2 may collect and distribute crates from the rooms 1. The central handling unit 3 is configured as described throughout the specification.

Fig. 5 is a further example of a linear configuration according to an example of the present disclosure. The system according to the example comprises two rooms 1 each being subdivided in compartments 11 and lanes 12 for storing the crates. The system further comprises a collection system 2, also referred to as logistics aisle, having at least two collection system devices 21 for connecting the rooms 1 with the central handling unit 3 comprising the different processing stations 5, 6, 7, 8. A connection station 4 (not shown in the picture) may connect the collection system 2 with the central handling unit 3. In this example, the collection system device 21 may act as the central handling unit 3 collecting and distributing crates to the respective processing stations 5, 6, 7, 8 directly. The processing stations 5, 6, 7, 8 may be located adjacent to the rooms 1 in another part of the facility shown on the right side of Fig. 5. It may be preferable, however, that the filling station 5 for providing (additional) feed to the crates is located close to the rooms 1 in order to ensure short paths of the crates when refeeding during a rearing process is necessary. The filling station 5 may also be located in the other part of the system or an additional filling station 5 may be provided in the other part of the system. There, a harvesting station 7 for further processing of the insects, a washing station 6 for washing the crates, a dosing station 5 for providing feed and/or insects to the crates and a rejection station 8 for rejection of crates that fail to meet the quality requirements may be provided as described throughout the specification.

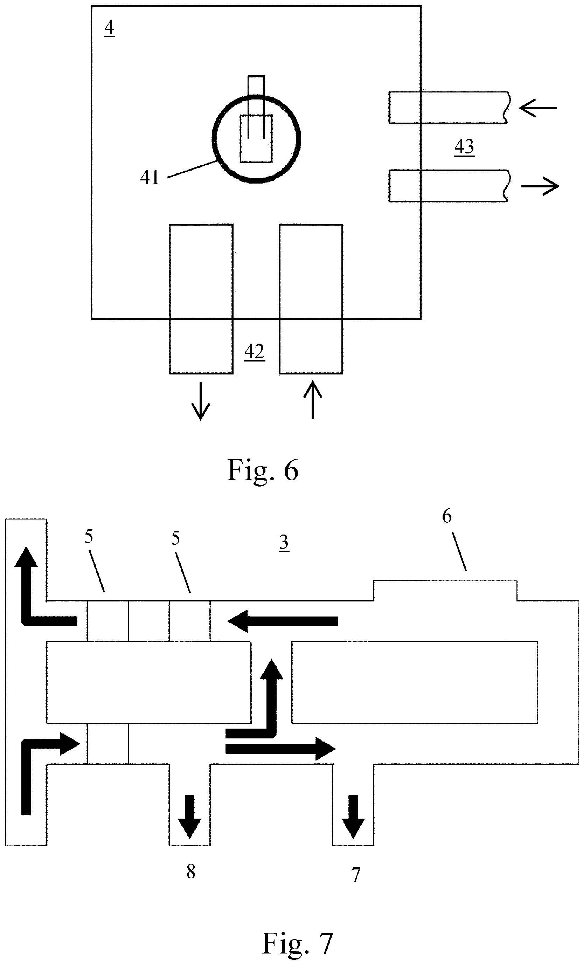

Fig. 6 is an exemplary depiction of a connection station 4 used in the present disclosure. The arrows indicate the movement direction of crates in the system. Reference numeral 42 indicates the part of the connection station 4 where the crates coming from the room 1 / compartment 11 / lane 12 arrive and are transported back from the connection station 4 to the room 1 / compartment 11 / lane 12. Reference numeral 43 on the other hand indicates transport from and to the processing stations 5, 6, 7, 8.

The crates are transported from the room 1 to the connection station 4. This can be done, e.g. by a collection system device 21 or manually. Examples are given throughout the specification. In case pallets are used, the connection station 4 may also comprise a de-palletiser or destacker in order to singularise the crates. The connection station 4 may also comprise a connection device 41 such as a robot arm for moving crates. The connection device 41 can move crates coming from the rooms 1 (section 42) to the processing stations 5, 6, 7, 8 (section 43) and vice versa. Thus, the processing stations may directly be connected to the rooms. The connection station 4 may be supported by manual intervention, e.g. using manual pallet trucks to move crates or stacks of crates. At reference numeral 43, e.g. a central handling unit 3 according to Fig. 7 may be connected.

Fig. 7 shows an example of a central handling unit 3 having a conveyor belt system or similar for transporting the crates. The movement direction is indicated by the arrows. The crates entering the central handling unit 3 may be checked whether the insects are ready for harvest or whether contamination or other unexpected occurrences lead to dismissal of the insect batch. In this case, the crate is moved to the rejection station 8 where the insects are removed from the crate. The respective crate may then be moved back to the central handling unit 3 where it is washed at the washing station 6 and refilled with feed and/or insects at the filling station 5. In case the insects are ready for being processed, the crate is moved to the harvesting station 7. Again, the emptied crate may then be recirculated, washed and filled with insects and/or feed. If the crates are removed from the rearing room 1, compartment 11 or lane 12 in order to provide additional feedstock, the crate may directly pass to the respective station 5 as indicated by the bended arrow without passing the harvesting station 7 and the washing station 6. The crates are, after the necessary actions are performed, transported back to the room 1, compartment 11 or lane 12.

The central handling unit 3 according to the disclosure may also comprise stackers and destackers for stacking and destacking or isolating of crates. Furthermore, the crates may be turned during transportation, e.g. for facilitating emptying or cleaning of the crates. Furthermore, a dryer may be provided for drying the crates after the washing.

With this system, a regular output of insects may be provided, since the central handling unit 3 is capable of automatically and centrally managing the crates according to the type and state of the insects. Thus, with different climate conditions and/or feedstock, depending on the content of the respective crate, a quasi-continuous rearing output may be provided by circulating the crates. Crates may also be inspected in the central handling unit using an automated inspection camera to determine the status and rearing stage of the insects within.

If deemed necessary after a rearing cycle or in case of contamination of a compartment 11 and/or room 1, the crates as well as the lanes 12 and/or compartments 11 and/or rooms 1 may be cleaned from larvae, residue, contaminants or feed. Empty rooms and/or compartments and/or lanes might be also needed for other operational actions like maintenance. Therefore, swap areas are provided as shown in the example Fig. 8. Swap areas can be any storage area suitable to accommodate one or more crates of insects. According to this example, one compartment 11, i.e. two lanes 12, are designated as a swap area. The compartments 11 form part of a room 1 as shown for example in Fig. 2 and explained above. In Fig. 8 a), the upper compartment 11 which is at the moment filled with crates needs to be cleaned, the swap area is empty. The crates are then transported to the swap area by any suitable transport means, preferably by the crate collection system device 21 (see Fig. 8b)). For example, the collection system or automated guided vehicles as described above may be used. When the compartment 11 to be cleaned is empty, it may be automatically or manually cleaned (Fig. 8 c)). Thereafter, the crates are transported back into the cleaned area leaving the swap area empty again and ready to be filled with crates as shown in Fig. 8d). The swap area thus serves as a temporary storage area or parking lot for crates. The swap area may be climatised according to the requirements of the stored crates. In other words, it may be designed as any of the other compartments 11. The swap area may be provided in one of the rooms 1 and/or adjacent to the rooms 1 and/or in the reach of the crate collection devices. Furthermore, the swap areas may also be filled with empty crates for storage if necessary. Swap areas may be used when cleaning of other compartments 11 or rooms 1 which is typically performed every ten days to prevent fly emergence and at least twice a year for a full cleaning. Furthermore, the emptied rooms 1 or compartments 11 may be subject to maintenance of the rooms/compartments themselves or e.g. the climate system. Utilisation of the swap area(s) may have a separate timing to harvesting and feeding or might be performed at the same time as harvesting and feeding. The swap area may comprise one or more lanes 12, one or more compartments 11 or one or more rooms 1. Preferably, one compartment 11 is used as a swap lane. Several swap areas may be provided in one facility.

This may further help providing the functionality of quasi-continuously or regularly outputting insects. With the swap areas, the downtime of compartment 11 or lanes 12 may be minimised or in other words the crate using time may be optimised.

The term continuously may be understood as performing harvesting in regular intervals. The intervals may be fixed or may be adapted according to the requirements of the insects. In other words, the system and method proposed herein may ensure a constant product flow. In the different example embodiments, insect harvesting is for example performed 14 hours a day or 7 hours a day, wherein the remaining 10 hours or 17 hours, respectively, can be used for cleaning or no movement of crates is performed. Depending on the facility, insect type and other circumstances, the harvesting times may vary. All embodiments of the present disclosure may be employed regardless of the rearing cycles.

According to an example of the present disclosure, a system or facility for rearing insects comprises a plurality of crates for storing the insects which are reared. The crates are preferably rectangular and stackable and set up to be picked up by e.g. a forklift or any automated device. The following example will be explained using crate measuring 800 x 600 x 290 mm, but the present disclosure is not limited to these dimensions and shapes. The crates preferably allow air circulation for providing optimal climate conditions to the insects. Furthermore, the crates are filled with feedstock and insect larvae, wherein the feedstock is varied according to the reared insects. The filling can be performed at a filling station or dosing station 5 which is configured to provide both insects and feed or at a plurality of stations, one providing insects while the rest provide feed. A mixture of insects and feed may also be provided at one of the stations. The station for providing additional feedstock and/or the station for providing insects may also be integrated into a connection station 4. Additionally, a weighting station may be provided to determine the weight of the crates and/or the weight of the added insects and/or feedstock. The weighting station or stations may be provided separately or may be included in any of the processing stations.

The filled crates are then stored in a rearing room 1 which is subdivided in compartments 11 and lanes 12 as explained above in conjunction with the figures. The system according to the present disclosure comprises at least one rearing room 1. The compartments 11 or rooms 1 may be climatised differently depending on the type of insect and development stage. Thus, a rearing facility is capable of simultaneously rearing different kinds of insects and different development stages while providing a continuous output.

At the end of a rearing cycle, the crates are transported to a harvesting station 7, where the insects are unloaded and processed further. In addition, a station for removing the insect skins, e.g. for mealworms, may be provided.

After emptying or harvesting, respectively, at the harvesting station 7, the crates are transported to a washing station 6, where residues are removed from the crates and the crates are cleaned and prepared to be filled again. The crates are therefore used in a closed loop inside the system. However, at any point, crates may be manually removed and/or added to the system, particularly the central handling unit.

Depending on the type of insect, the crates may, during a rearing cycle, be transported to a filling station in fixed intervals or when deemed necessary for providing additional feedstock. The climate conditions as well as the development stage of the insects may be monitored during rearing.

In order to transport the crates between the processing stations, i.e. the filling station 5, the washing station 6, the harvesting station 7 and the rejection station 8, a central handling unit 3 is provided. The central handling unit 3 is accessible from all of the rooms 1 and connects with all of the processing stations 5, 6, 7, 8 and is thus capable of centrally managing the complete rearing facility having a plurality of rooms 1. A connection station 4 may be needed to connect the central handling unit 3 to the collection system 2.The central handling unit 3 may be designed as a loop and may be operated uni- or bidirectionally. The connection between the processing stations 5, 6, 7, 8 within the central handling unit 3 and/or the collection system 2 may be fixed. In the case automated guided vehicles are used, they may also freely move inside the facility or move on previously set tracks. All processing stations 5, 6, 7, 8 can be accessed or bypassed by the central handling unit 3 as also shown in Fig. 2. The central handling unit 3 uses any kind of logistics system such as conveyor belts with a shuttle satellite system or automated guided vehicles.

The system also comprises a controller which controls rearing of insects in individual crates or sets of crates. In particular, the controller controls the central handling unit 3. The controller may further control at least one of the processing stations 5, 6, 7, 8 and/or the connection station 4. The controller may also control and/or monitor climate conditions inside the rooms 1 and/or compartments 11. The controller may therefore also be configured such that the system provides a regular output of reared insects. Also, a control system comprising one or a plurality of controllers may be provided for controlling at least one of the central handling unit 3, the processing stations 5, 6, 7, 8, the collection system 2, the connection station 4 or the collection system devices 21.

The example described above can also implement the features described in conjunction with all figures and vice versa.

A central handling unit 3 as described above and as preferred may handle up to thirty-six rooms 1. An insect rearing facility may contain a plurality of central handling units 3. A room 1 may consist of two (2) to seventy-two (72) compartments 11. Each compartment 11 may have between two (2) and twenty-four (24) lanes 12. Each lane 12 may have between two (2) and sixty-four (64) crate stacks. Depending on the height of the crate stacks, each compartment 11 may for example house hundred twenty (120) to twenty-five thousand (25'000) crates. It is understood that the crate numbers per compartment may vary with different crate sizes. If separate feeding stations are provided, the number of feeding stations may correspond to the number of rooms 1 or may be any other number sufficient for providing feed to the insects. As noted above, the feeding stations may also be partially or completely integrated into connection stations 4 or filling stations 5. The given numbers are purely exemplary and not to be understood as limiting.

The following will give an overview of exemplary configurations according to the present disclosure. These configurations are compatible with all examples described above.

According to the disclosure, crates may be stored in a room on the ground, on pallets, on support elements with or without pallets, in a rack system or other suitable means. The crates may be stacked and the stacked crates may be stored in lanes. The room may be climatised. Moreover, the compartments, lanes or even crates may be singularly climatised according to the requirements of the reared insects. The rooms, compartments or lanes may have insects of different life stages.

The crate collection system with multiple crate collection devices interconnects rooms, processing stations, the central handling unit and/or swap areas. Crate collection devices such as the collection system devices describe above may move freely or on fixed pathways. Freely moving devices preferably are automated guided vehicles (AGVs). Fixed pathways may e.g. be confined by rails or other means guiding a shuttle-satellite system, an automated storage and retrieval system (AS/RS), or conveyor belt system. The crate collection devices can be used to transport crates to a swap area, to a non-central feed dosing unit or inspection station or the central handling unit.

The connection station (also referred to as "connection") interconnects rooms, processing stations and/or the central handling unit. The connection may be a connection formed by collection system devices or other means. The connection may be performed freely or on fixed pathways. Freely moving devices preferably are AGVs or pallet trolleys. Fixed pathways may be confined by rails, guide means or similar. The connection can be provided as a stationary system such as a conveyor belt or hanger system, a shuttle system, a pusher system or a robotic arm. The connection may also include stacking and de-stacking of the crates by a destacker, stacker or a robotic arm.

The central handling unit may be a separated connecting unit or comprise at least one of the following: a harvesting station for unloading insects from the crates followed by separating insects from frass, a dosing station for filling insects and/or feed to the crates, a crate washer (and possibly dryer) of crates, weighing means for determining the weight of crates, observation means such as a camera, a crate (de-)stacker, a reject line or rejection station, a bypass for one or more particular processing stations or a station for removal of frass. The dosing station may be separately provided. Automated transport of crates may be performed by AGVs or a conveyor belt system.

Depending on the configuration, the following options are possible. The elements have been described in detail above.

When using stacks of crates without pallets and a shuttle-satellite for crate transport, de-stacking may be performed before, within or after the connection station. When using an AGV for crate transport, de-stacking may be performed before the connection station or the connection station may be omitted and the AGVs may transport the crates to de-stacking. Subsequently, the crates are moved to the central handling unit.

When using stacks on pallets in an AS/RS, the following configurations are possible. The crates may be de-palletised, de-stacked and given to the connection station and subsequently the central handling unit. Alternatively, the crates may be de-palletised, given to the connection station, be de-stacked and then moved to the central handling unit. As a further alternative, the complete pallets may be transported to the connection station and afterwards de-palletised, de-stacked and brought to the central handling unit.

The same procedures as with the AS/RS may apply to stacks of crates and AGVs for transportation.

In case single big boxes for rearing insects are used, transportation from the room to the central handling unit may directly performed via shuttle satellites, AS/RS or AGVs.

The AGVs described herein may e.g. be a pallet truck, a move-lift truck or a wheeled pallet.

The present disclosure also relates to a corresponding method for rearing insects or a method for operating an insect rearing system, preferably as described above.

The system of a facility thus consists of a plurality of rooms 1 as well as processing stations 5, 6, 7, 8 with respective actions such as filling, feeding, tipping, cleaning and re-batching. By the central handling unit 3, a constant product flow is ensured. The crates do not leave the system during normal operation and rather form a rearing loop. The rearing cycles vary according to the reared insects. Black soldier flies may have a rearing cycle of 4 to 16 days, while mealworms may take up to 45 days until they are ready for processing.

If automated guided vehicles (AGVs) or shuttle satellites are used in the central handling unit 3, the number thereof may be adapted to the number of rooms 1. The crates or stacks of crates may also be positioned on pallets which are configured to be picked up by the collection system 2, which transfers it to the central handling unit 3.

The examples above refer to the division in rooms, compartments and lanes. However, any further subdivision or no subdivision at all is also encompassed by the present disclosure and may be used with the system and method presented herein.

Preferably, the crates are stored as stacks. Furthermore, stackers and de-stackers may be provided in the system to form stacks of crates or isolate crates depending on the stage of processing. In particular, for feeding, adding insects, harvesting, rejecting and washing, the crates need to be de-stacked. Alternatively, pallets may be used.

With the system and method as described above it is possible to provide a constant product flow by having a central handling unit or central harvesting unit which centrally manages the crates according to the stored insects and/or development stages. Multiple rooms can be connected with the central handling unit and the facility allows cleaning and maintaining of areas after or during a rearing cycle without halting the production. By providing at least two collection devices interconnecting the processing stations and connecting the processing stations with the room(s), a regular or continuous output of insects may be ensured. The subdivision in compartments also prevents cross contamination between compartments or rooms. If one of the processing stations fails or requires maintenance, crates may also be redirected to other processing stations by the central handling unit.

Other aspects, features, and advantages will be apparent from the summary above, as well as from the description that follows, including the figures and the claims.

While the invention has been illustrated and described in detail in the drawings and foregoing description, such illustration and description are to be considered illustrative or exemplary and not restrictive. It will be understood that changes and modifications may be made by those of ordinary skill within the scope of the following claims. In particular, the present invention covers further embodiments with any combination of features from different embodiments described above and below.

Furthermore, in the claims the word "comprising" does not exclude other elements or steps, and the indefinite article "a" or "an" does not exclude a plurality. A single unit may fulfil the functions of several features recited in the claims. The terms "essentially", "about", "approximately" and the like in connection with an attribute or a value particularly also define exactly the attribute or exactly the value, respectively. Any reference signs in the claims should not be construed as limiting the scope.

### List of reference signs

- 1: (Rearing) Room
- 11: Compartments
- 12: Lanes
- 2: Collection System
- 21: Collection System Device / Crate Collection Device
- 3: Central Handling Unit / Central Harvesting Unit
- 31: Bypass / Re-batch
- 4: Connection Station
- 41: Connection Device
- 42: From/To Room
- 43: To/From Processing Stations
- 5: Filling Station / Dosing station
- 6: Washing Station
- 7: Harvesting Station
- 8: Rejection Station

## Claims

1. System for rearing insects, comprising
at least one room (1) for storing a plurality of crates for storing insects to be reared,
a dosing station (5) for filling insects and/or feed to the crates,
an automated central harvesting unit (3) including a harvesting station (7) for unloading the insects from the crates for subsequent processing of the insects and/or a plurality of further processing stations,
a crate collection system for transferring the crates between the at least one room (1) and the central harvesting unit (3) comprising
a plurality of crate collection devices for at least collecting crates in or from the at least one room (1), and
a connection for connecting the at least one room (1) and the central harvesting unit (3),
wherein the crates are used in a continuous loop in the system.

2. The system according to claim 1, wherein of the at least one room (1) is subdivided in separate compartments (11).

3. The system according to claim 1 or 2, further comprising a climate system adapted to individually control the climate conditions for the at least one room (1) and/or each compartment (11).

4. The system according to any one of the preceding claims, wherein the central harvesting unit (3) further includes, as a further processing station, a washing station (6) for cleaning and possibly drying empty crates and/or a weighing station for measuring the weight of a crate.

5. The system according to any one of the preceding claims, wherein the central harvesting unit (3) comprises a bypass for allowing an individual crate on the central harvesting unit (3) to bypass a processing station.

6. The system according to any one of the preceding claims, ,
wherein the connection is provided by the crate collection devices of the collection system which directly or indirectly connect the at least one room (1) with at least one of the processing stations of the central harvesting unit (3), and/or
wherein the connection between the at least one room (1) and/or central harvesting unit (3) and/or processing stations is stationary and/or
wherein the connection between the at least one room (1) and/or central harvesting unit (3) and/or processing stations is mobile, as the crate collection system comprising automated guided vehicles for transporting individual crates or stacks of crates,
wherein preferably the transporting or the transferring of the crates by or along the connection is automated.

7. The system according to any one of the preceding claims, further comprising
a destacker for destacking stacks of crates into individual crates and/or
a stacker for stacking individual crates into stacks and/or
a depalletizer for unloading crates or stacks of crates from a pallet and/or
a palletizer for stacking crates or stacks of crates onto a pallet.

8. The system according to any one of the preceding claims, further comprising a controller for controlling rearing of insects in individual crates or sets of crates by automatically controlled transporting of the crates or sets of crates between the at least one room (1) and the central harvesting unit and/or processing stations, the controller being configured such that the system provides for a regular output of reared insects.

9. A system for rearing insects, comprising
at least one room (1), at least one compartment of a room and/or at least one lane for storing a plurality of crates for storing the insects to be reared,
at least one swap area, leaving space for crates to be temporarily stored, and
a collection system (2) for transporting the crates between rooms, compartments (11), and/or lanes and the swap area or vice versa.

10. The system according to claim 9, wherein the swap area is provided in at least one of the compartments (11) and/or adjacent to the at least one rooms (1) and/or is within the reach of the crate collection system (2).

11. The system according to claim 9 or 10, wherein the swap area has a size for storing crates of an entire lane, of a compartment and/or of a room.

12. Method for operating an insect rearing system, in particular the system according to any one of the preceding claims, the system comprising at least one room (1) for storing a plurality of crates for rearing the insects, a dosing station (4, 5, 8) for filling insects and/or feed to the crates, a central crate harvesting unit (3) including a harvesting station (7) for unloading the insects from the crates for subsequent processing of the insects and/or comprising a plurality of further processing stations, and a crate collection system for transferring the crates between the at least one room and the central harvesting unit comprising a plurality of crate collection devices for collecting crates in or from the at least one room (1), the crates being transferred between the at least one room (1) and the central harvesting unit (3) via a connection, by automatically individually controlling a path of the crates through the rearing system to
fill insects and/or feed to the crates,
store the crates in the at least one room (1), thereby rearing the insects, and
harvest the insects by unloading the insects and the feedstock residue from the crates for subsequent processing of the insects,
wherein the path of the plurality of crates through the system is controlled in such a way as to provide a regular output of reared insects.

13. The method of claim 12, wherein the crates are used inside the system in a continuous loop.

14. The method of claim 12 or 13, further comprising the step of washing and drying the crates and/or weighing after harvesting the insects.

15. The method of any one of claims 12 to 14, wherein the crates are automatically transported between the processing stations performing the respective method steps and/or between the at least one room (1) and the central harvesting unit (3) and/or between the plurality of crate collection devices by using a connection such as conveyor belts or automated guided vehicles.
